(19) Europäisches Patentamt
European Patent Office
Office européen des brevets

(11) Veröffentlichungsnummer: **0 029 542**
**B1**

(12) # EUROPÄISCHE PATENTSCHRIFT

(45) Veröffentlichungstag der Patentschrift: **18.07.84**

(21) Anmeldenummer: **80106909.7**

(22) Anmeldetag: **08.11.80**

(51) Int. Cl.³: **C 07 D 233/60**, C 07 D 303/04, C 07 D 303/08, C 07 D 303/22, A 01 N 43/50 // C07D301/00, C07C49/76, C07C49/807

(54) **Hydroxybutyl-Imidazol-Derivate, Verfahren zu ihrer Herstellung, zu letzterer benötigte Zwischenprodukte sowie ihre Verwendung als Fungizide.**

(30) Priorität: **21.11.79 DE 2946956**

(43) Veröffentlichungstag der Anmeldung: **03.06.81 Patentblatt 81/22**

(45) Bekanntmachung des Hinweises auf die Patenterteilung: **18.07.84 Patentblatt 84/29**

(84) Benannte Vertragsstaaten: **BE CH DE FR GB IT LI NL**

(56) Entgegenhaltungen:
**EP - A - 0 031 883**
**DE - A - 2 205 732**
**DE - A - 2 623 129**
**DE - A - 2 736 122**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Regel, Erik, Ing. grad., Bergerheide 72a, D-5600 Wuppertal 1 (DE)**
Erfinder: **Büchel, Karl-Heinz, Prof. Dr., Dabringhausener Strasse 42, D-5093 Burscheid (DE)**
Erfinder: **Brandes, Wilhelm, Dr., Eichendorffstrasse 3, D-5653 Leichlingen (DE)**
Erfinder: **Frohberger, Paul-Ernst, Dr., Willi-Baumeister-Strasse 5, D-5090 Leverkusen (DE)**
Erfinder: **Paul, Volker, Dr., Ahornstrasse 5, D-5650 Solingen (DE)**

**EP 0 029 542 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue Hydroxybutylimidazol-Derivate, ein Verfahren zu ihrer Herstellung sowie ihre Verwendung als Fungizide. Sie betrifft weiterhin neue Oxirane als Zwischenprodukte zur Herstellung dieser Verbindungen.

Es ist bereits bekanntgeworden, daß substituierte 1-Phenyl-2-imidazolyl-1-ethanole, wie z. B. 1-Halogenphenyl-2-imidazolyl-1-ethanole, zur Herstellung von fungizid wirksamen 1-($\beta$-Phenyl)-ethyl-imidazol-ethern verwendet werden können (vergleiche US-Patentschrift 3 717 655). Die fungizide Wirksamkeit dieser Zwischenprodukte ist jedoch, insbesondere bei niedrigen Aufwandmengen und -konzentrationen, nicht immer voll befriedigend.

Es wurden neue Hydroxybutyl-imidazol-Derivate der allgemeinen Formel

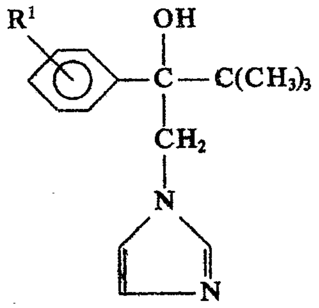

(I)

in welcher

$R^1$ für gegebenenfalls durch Fluor, Chlor, Brom, Alkyl und Alkoxy mit jeweils 1 bis 2 Kohlenstoffatomen substituiertes Phenyl steht

und deren physiologisch verträglichen Säureadditions-Salze gefunden.

Weiterhin wurde gefunden, daß man die Hydroxybutyl-imidazol-Derivate der Formel (I) erhält, wenn man Oxirane der Formel

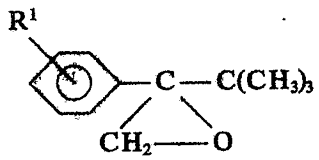

(II)

in welcher

$R^1$ die oben angegebene Bedeutung hat,

mit Imidazol in Gegenwart eines Alkalialkoholats und in Gegenwart eines Verdünnungsmittels umsetzt.

Weiterhin können die erfindungsgemäß erhältlichen Hydroxybutyl-imidazol-Derivate der Formel (I) durch Umsetzen mit Säuren in die Salze überführt werden.

Die neuen Hydroxybutyl-imidazol-Derivate weisen starke fungizide Eigenschaften auf. Dabei zeigen überraschenderweise die erfindungsgemäßen Verbindungen eine bessere fungizide Wirksamkeit als die aus dem Stand der Technik bekannten 1-Halogenphenyl-2-imidazolyl-1-ethanole, welche chemisch naheliegende Verbindungen sind. Die erfindungsgemäßen Stoffe stellen somit eine Bereicherung der Technik dar.

Die erfindungsgemäßen Hydroxybutyl-imidazol-Derivate sind durch die Formel (I) allgemein definiert.

Bevorzugt sind diejenigen Verbindungen der Formel (I), in denen $R^1$ für Phenyl steht, welches gegebenenfalls einfach oder zweifach durch Chlor, Brom, Fluor, Methyl oder Methoxy substituiert sein kann.

Im einzelnen seien außer den bei den Herstellungsbeispielen genannten Verbindungen die folgenden Verbindungen der allgemeinen Formel (I) genannt:

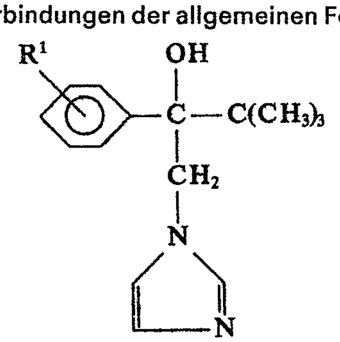

(I)

| $R^1$ |
|---|
| 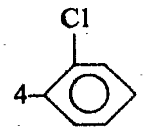 |
| 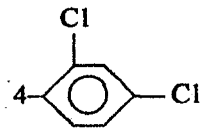 |
| 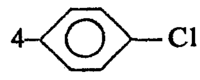 |
| 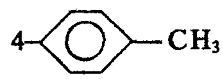 |
| 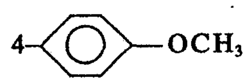 |

Verwendet man beispielsweise 2-(4-Biphenylyl)-2-tert.-butyl-oxiran, Imidazol und Natriummethylat als Ausgangsstoffe, so kann der Reaktionsablauf durch das folgende Formelschema wiedergegeben werden:

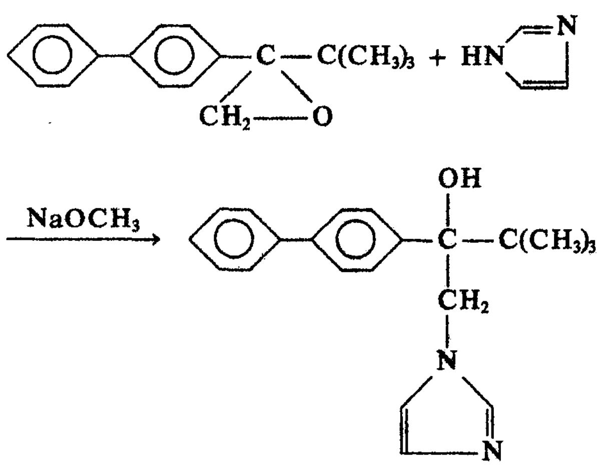


Die bei der Durchführung des erfindungsgemäßen Verfahrens als Ausgangsstoffe zu verwendenden Oxirane sind durch die Formel (II) allgemein definiert. In dieser Formel steht $R^1$ vorzugsweise für die Reste, die bereits im Zusammenhang mit der Beschreibung der erfindungsgemäßen Stoffe der Formel (I) vorzugsweise für diesen Substituenten genannt wurden.

Die Oxirane der Formel (II) sind noch nicht bekannt. Sie werden erhalten, indem man entsprechende Ketone der Formel

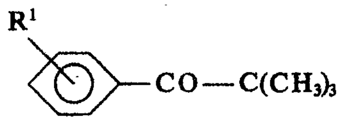 (III)

in welcher
$R^1$ die oben angegebene Bedeutung hat,

α) mit Dimethyloxosulfonium-methylid der Formel

$$(CH_3)_2\overset{\delta+\delta-}{SOCH_2} \quad (IV)$$

in an sich bekannter Weise in Gegenwart eines Verdünnungsmittels, wie beispielsweise Dimethylsulfoxid, bei Temperaturen zwischen 20 und 80°C umsetzt (vergleiche hierzu auch die Angaben in J. Am. Chem. Soc. 87, 1363—1364 [1965]), oder

β) mit Trimethylsulfonium-methylsulfat der Formel

$$[(CH_3)_3S^{\oplus}]CH_3SO_4^{\ominus} \quad (V)$$

in an sich bekannter Weise in Gegenwart eines Zweiphasensystems und gegebenenfalls in Gegenwart eines Phasentransferkatalysators bei Temperaturen zwischen 0 und 100°C umsetzt (vergleiche auch die Angaben in Heterocycles 8, 397 [1977]).

Die Ketone der Formel (III) sind allgemein bekannte Verbindungen der organischen Chemie.

Das Dimethyloxosulfonium-methylid der Formel (IV) ist ebenfalls bekannt und wird in situ eingesetzt, indem man Trimethyloxosulfoniumjodid mit Natriumhydrid (vergleiche auch die o. g. Literaturstelle) bzw. Natriumamid umsetzt. Das Trimethylsulfonium-methylsulfat der Formel (V) ist auch bekannt und wird in situ eingesetzt, indem man Dimethylsulfid mit Dimethylsulfat umsetzt (vergleiche auch die o. g. Literaturstelle).

Die erfindungsgemäße Umsetzung wird in Gegenwart eines Alkalialkoholats durchgeführt. Hierzu gehören vorzugsweise die Methylate und Ethylate von Natrium und Kalium.

Für die erfindungsgemäße Umsetzung kommen als Verdünnungsmittel vorzugsweise inerte organische Lösungsmittel in Frage. Hierzu gehören vorzugsweise Nitrile, wie insbesondere Acetonitril; aromatische Kohlenwasserstoffe, wie Benzol; Toluol und Dichlorbenzol; Formamide, wie insbesondere Dimethylformamid; halogenierte Kohlenwasserstoffe, wie Methylenchlorid oder Tetrachlorkohlenstoff; sowie Hexamethylphosphortriamid.

Die Reaktionstemperaturen können bei der Durchführung des erfindungsgemäßen Verfahrens in einem größeren Bereich variiert werden. Im allgemeinen arbeitet man zwischen etwa 30 bis 150°C, vorzugsweise zwischen 30°C und 100°C.

Bei der Durchführung des erfindungsgemäßen Verfahrens setzt man auf 1 Mol Oxiran der Formel (II) vorzugsweise 1 bis 3 Mol Imidazol und 1 bis 2 Mol Alkalialkoholat ein. Die Isolierung der Endprodukte erfolgt in allgemein üblicher Weise.

In einer bevorzugten Ausführungsform werden die gemäß Verfahren (α) oder (β) erhaltenen Oxirane der Formel (II) ohne Isolierung direkt weiter umgesetzt.

Die erfindungsgemäßen Verbindungen der Formel (I) können auch erhalten werden, wenn man z. B. entsprechende Imidazolylmethyl-phenyl-ketone in bekannter Art und Weise mit tert.-Butyl-Magnesiumbromid umsetzt oder wenn man die entsprechenden 1-Halogen-2-hydroxy-2-phenyl-3,3-dimethylbutane in bekannter Art und Weise mit Imidazol umsetzt.

Zur Herstellung von Säureadditionssalzen der Verbindungen der Formel (I) kommen alle physiologisch verträglichen Säuren in Frage. Hierzu gehören vorzugsweise die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure sowie Sulfonsäuren, wie z. B. p-Toluolsulfonsäure und 1,5-Naphthalindisulfonsäure.

Die Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg

zur Bekämpfung von Podosphaera- und Erysiphe-Arten eingesetzt werden, wie gegen den Erreger des echten Apfelmehltaus (Podosphaera leucotricha) und gegen den Erreger des echten Gurkenmehltaus (Erysiphe cichoracearum). Gute Erfolge werden auch bei der Bekämpfung von Getreidekrankheiten, wie insbesondere Getreidemehltau und Getreiderost, erzielt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate, angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

## Herstellungsbeispiele

### Beispiel 1

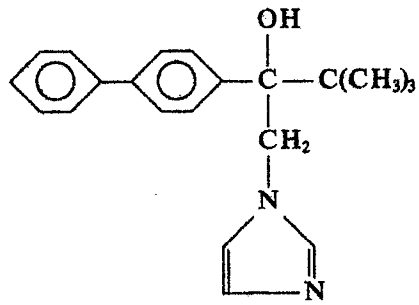

Eine Lösung von 22,2 g (0,088 Mol) 2-(4-Biphenylyl)-2-(tert.-butyl)-oxiran in 150 ml Dimethylformamid wird in eine Lösung von 6,4 g (0,117 Mol) Natriummethylat und 13,2 g (0,2 Mol) Imidazol in 40 ml Methylalkohol getropft. Nach 6stündigem Erwärmen auf 80° C wird das Gemisch im Vakuum eingeengt, auf 3000 ml Wasser gegeben, die ausgeschiedene Kristallmasse in Methylenchlorid gelöst und die Lösung mit Wasser gewaschen. Die Lösung wird über Natriumsulfat getrocknet und im Vakuum eingedampft. Die Kristalle werden mit Ether verrührt. Man erhält 20,5 g (73% der Theorie) 1-(4-Biphenylyl)-1-(tert.-butyl)-2-(imidazol-1-yl)-ethan-1-ol vom Schmelzpunkt 184° C.

Herstellung des Ausgangsproduktes
(neues Zwischenprodukt der Formel (II))

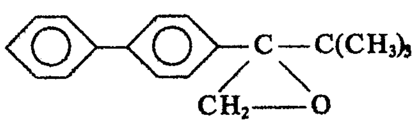

(II-1)

3,6 g (0,12 Mol) 80%iges Natriumhydrid und 26,4 g (0,12 Mol) Trimethyloxosulfoniumjodid werden unter Rühren tropfenweise mit 120 ml Dimethylsulfoxid versetzt. Anschließend wird eine Lösung von 23,8 g (0,1 Mol) 4-Biphenylyl-tert.-butyl-keton in 150 ml Dimethylsulfoxid unter Rühren zugetropft. Das Gemisch wird 3 Stunden auf 50° C erwärmt, danach werden unter Kühlen bei 20° C 500 ml Wasser zugetropft. Die ausgeschiedenen Kristalle werden in Chloroform gelöst, die Lösung wird mit Wasser gewaschen, die Chloroformlösung über Natriumsulfat getrocknet und am Rotationsverdampfer eingedampft. Die Kristallmasse wird mit Petrolether verrührt und abgesaugt. Man erhält 22,3 g (88% der Theorie) 2-(4-Biphenylyl)-2-(tert.-butyl)-oxiran vom Schmelzpunkt 78° C.

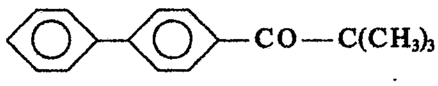

6 g (0,2 Mol) 80%iges Natriumhydrid werden in 100 ml Tetrahydrofuran vorgelegt. Unter Rühren werden 44,4 g (0,2 Mol) 4-Biphenylyl-isopropyl-keton eingetragen. Nach Abklingen der Gasentwicklung wird 3 Stunden auf 40° C erwärmt, dann werden 28,8 g (0,2 Mol) Methyljodid zugetropft. Nach 48 Stunden wird die Lösung filtriert und im Vakuum eingedampft; der Rückstand wird in Essigester gelöst, die Lösung mit Wasser gewaschen, über Natriumsulfat getrocknet und erneut im Vakuum eingedampft. Nach Verrühren der Kristallmasse mit Waschbenzin erhält man 40,5 g (85% der Theorie) 4-Biphenylyl-tert.-butyl-keton vom Schmelzpunkt 98° C.

### Beispiel 2

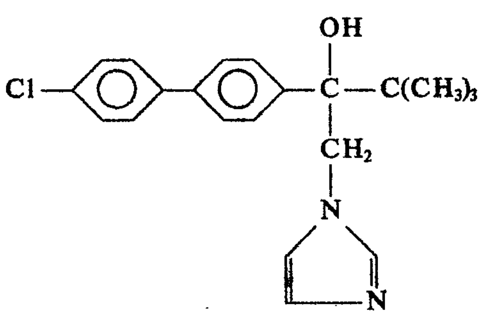

Entsprechend Beispiel 1 erhält man aus 5,6 g (0,104 Mol) Natriummethylat, 40 ml Methylalkohol, 12,2 g (0,18 Mol) Imidazol, 22,8 g (0,08 Mol) 2-(4-p-Chlorbiphenylyl)-2-(tert.-butyl)-oxiran und 150 ml Dimethylformamid 23,5 g (83% der Theorie) 1-(4-p-Chlorbiphenylyl)-1-(tert.-butyl)-2-(imidazol-1-yl)-ethan-1-ol vom Schmelzpunkt 164° C.

Herstellung des Ausgangsproduktes

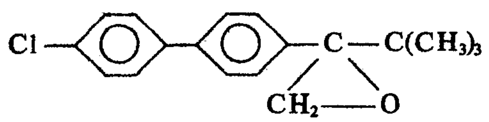

Entsprechend Beispiel 1 erhält man aus 3,6 g (0,12 Mol) Natriumhydrid (80%ig) 26,4 g (0,12 Mol) Trimethyloxosulfoniumjodid, 27,3 g (0,1 Mol) 4-p-Chlorbiphenylyl)-tert.-butyl-keton und 220 ml Dimethylsulfoxid nach Verrühren des Rohproduktes mit Diisopropylether 17,3 g (60% der Theorie) 2-(4-p-Chlorbiphenylyl)-2-(tert.-butyl)-oxiran vom Schmelzpunkt 118° C.

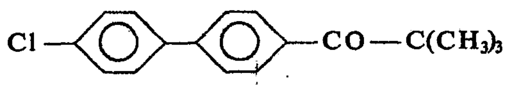

Entsprechend Beispiel 1 erhält man durch Umsetzung von 4-p-Chlorbiphenylyl-isopropyl-keton mit Methyljodid in 70%iger Ausbeute 4-p-Chlorbiphenylyl-tert.-butyl-keton vom Schmelzpunkt 99° C.

Anwendungsbeispiele

In den nachfolgenden Beispielen werden die nachstehend angegebenen Verbindungen als Vergleichssubstanzen eingesetzt:

(A) 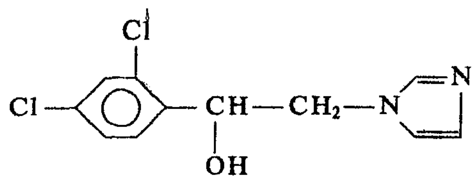

(B) 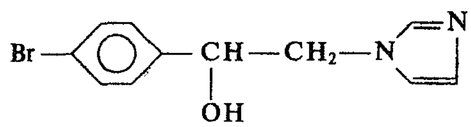

(C) 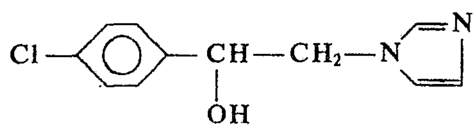

Beispiel A

Podosphaera-Test (Apfel)/protektiv

| | |
|---|---|
| Lösungsmittel: | 4,7 Gewichtsteile Aceton |
| Emulgator: | 0,3 Gewichtsteile Alkylarylpolyglykolether |
| Wasser: | 95,0 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20° C und einer relativen Luftfeuchtigkeit von 70% im Gewächshaus. Anschließend werden sie durch Bestäuben mit Konidien des Apfelmehltauerregers (Podosphaera leucotricha) inokuliert und in ein Gewächshaus mit einer Temperatur von 21 bis 23° C und einer relativen Luftfeuchtigkeit von 70% gebraucht.

10 Tage nach der Inokulation wird der Befall der Sämlinge bestimmt. Die erhaltenen Boniturwerte

werden in Prozent Befall umgerechnet. 0% bedeutet keinen Befall, 100% bedeutet, daß die Pflanzen vollständig befallen sind.

In diesem Test zeigen z. B. folgende Verbindungen eine sehr gute Wirkung, die derjenigen der aus dem Stand der Technik bekannten Verbindung (A) deutlich überlegen ist:

Verbindungen gemäß Herstellungsbeispielen 1 und 2.

Tabelle A

Podosphaera-Test/protektiv

| Wirkstoff | Befall in % bei einer Wirkstoffkonzentration von 0,001 % |
|---|---|
| 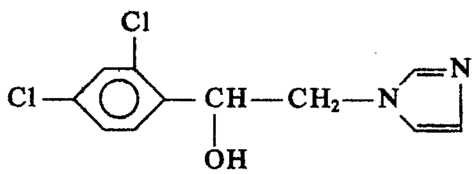 (A) (bekannt) | 100 |
| 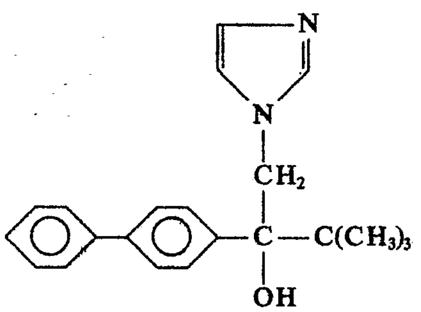 (1) | 67 |
| 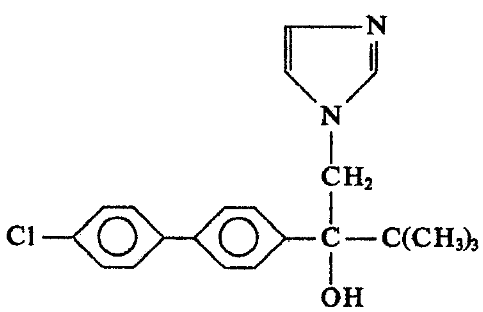 (2) | 54 |

Beispiel B

Erysiphe-Test (Gurken)/protektiv

| | |
|---|---|
| Lösungsmittel: | 4,7 Gewichtsteile Aceton |
| Emulgator: | 0,3 Gewichtsteile Alkyl-aryl-polyglykolether |
| Wasser: | 95,0 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoracearum bestäubt. Die Pflanzen werden

anschließend bei 23 bis 24°C und bei einer relativen Luftfeuchtigkeit von ca. 75% im Gewächshaus aufgestellt.

Nach 12 Stunden wird der Befall der Gurkenpflanzen bestimmt. Die erhaltenen Boniturwerte werden in Prozent Befall umgerechnet. 0% bedeutet keinen Befall, 100% bedeutet, daß die Pflanzen vollständig befallen sind.

Bei diesem Test zeigen z. B. die folgenden Verbindungen eine sehr gute Wirkung, die derjenigen der aus dem Stand der Technik bekannten Verbindungen (B) und (C) überlegen ist:

Verbindungen gemäß Herstellungsbeispielen 1 und 2.

**Tabelle B**

**Erysiphe-Test (Gurken)/protektiv**

| Wirkstoff | Befall in % bei einer Wirkstoffkonzentration von 0,005 % |
|---|---|
| 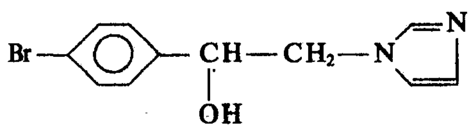 (B) (bekannt) | 100 |
| 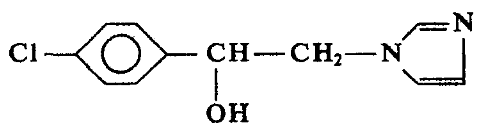 (C) (bekannt) | 71 |
| 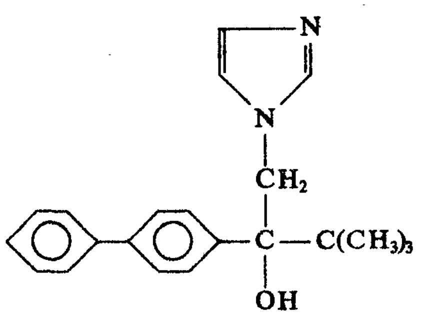 (1) | 15 |
| 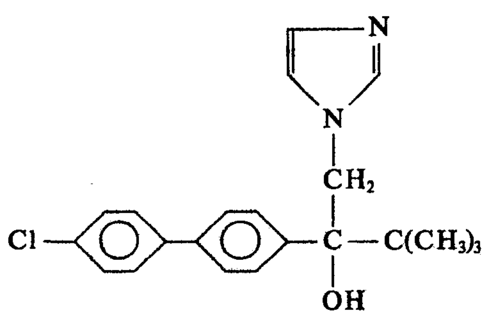 (2) | 16 |

Beispiel C

Sproßbehandlungs-Test/Getreidemehltau/protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykolether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var.hordei.

Nach 6 Tagen Verweilzeit der Pflanzen bei einer Temperatur von 21—22° C und einer Luftfeuchtigkeit von 80—90% wertet man den Besatz der Pflanzen mit Mehltaupusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Mehltaubefall ist.

Bei diesem Test zeigen z. B. die folgenden Verbindungen eine sehr gute Wirkung, die derjenigen der aus dem Stand der Technik bekannten Verbindung (B) überlegen ist:

Verbindungen gemäß Herstellungsbeispielen 1 und 2.

**Tabelle C**

**Sproßbehandlungs-Test/Getreidemehltau/protektiv**

| Wirkstoffe | Wirkstoffkonzentration in der Spritzbrühe in Gew.% | Befall in % der unbehandelten Kontrolle |
|---|---|---|
| 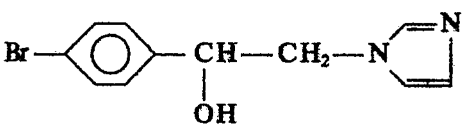 (B) (bekannt) | 0,025 | 50,0 |
| 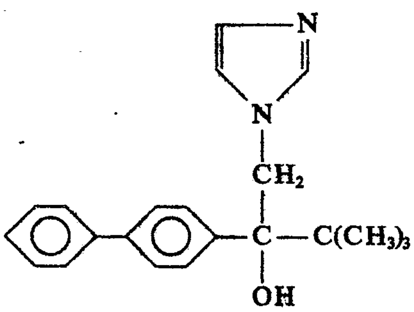 (1) | 0,025 | 12,5 |
| 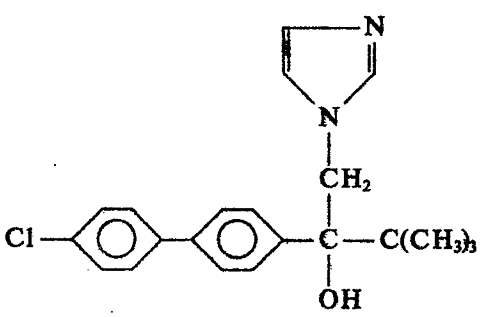 (2) | 0,025 | 25,0 |

## Beispiel D

Sproßbehandlungs-Test/Getreiderost/protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator Alkylaryl-polyglykolether

auf und gibt 975 Gewichtteile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von Puccinia recendita in 0,1%igem Wasseragar. Nach Antrocknen der Sporensuspension besprüht man die Weizenpflanzen mit der Wirkstoffzubereitung taufeucht und stellt sie zur Inkubation für 24 Stunden bei etwa 20°C und einer 100%igen Luftfeuchtigkeit in ein Gewächshaus.

Nach 10 Tagen Verweilzeit der Pflanzen bei einer Temperatur von 20°C und einer Luftfeuchtigkeit von 80—90% wertet man den Besatz der Pflanzen mit Rostpusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Rostbefall ist.

In diesem Test zeigen z. B. die folgenden Verbindungen eine sehr gute Wirkung, die derjenigen der aus dem Stand der Technik bekannten Verbindung (B) überlegen ist:

Verbindung gemäß Herstellungsbeispiel 2.

**Tabelle D**

**Sproßbehandlungs-Test/Getreiderost/protektiv**

| Wirkstoffe | Wirkstoffkonzentration in der Spritzbrühe in Gew.% | Befall in % der unbehandelten Kontrolle |
|---|---|---|
| 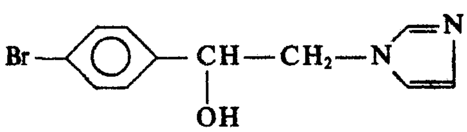 (B) (bekannt) | 0,025 | 100 |
| 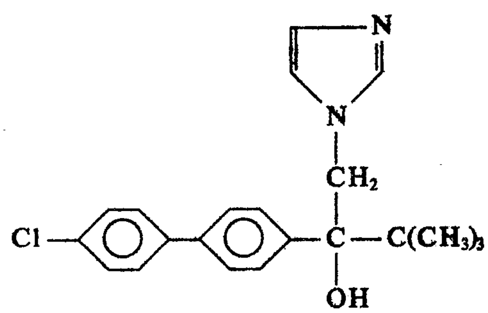 (2) | 0,025 | 50,0 |

Beispiel E

Myzelwachstums-Test

Verwendeter Nährboden:

| | |
|---|---|
| 20 Gewichtsteile | Agar-Agar |
| 200 Gewichtsteile | Kartoffeldekokt |
| 5 Gewichtsteile | Malz |
| 15 Gewichtsteile | Dextrose |
| 5 Gewichtsteile | Pepton |
| 2 Gewichtsteile | $Na_2HPO_4$ |
| 0,3 Gewichtsteile | $Ca(NO_3)_2$ |

Verhältnis von Lösungsmittelgemisch zum Nährboden:

| | |
|---|---|
| 2 Gewichtsteile | Lösungsmittelgemisch |
| 100 Gewichtsteile | Agarnährboden |

Zusammensetzung Lösungsmittelgemisch:

0,19 Gewichtsteile DMF oder Aceton
0,01 Gewichtsteile Emulgator Alkylarylpolyglykolether
1,80 Gewichtsteile Wasser
2 Gewichtsteile Lösungsmittelgemisch

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrieschalen mit einem Durchmesser von 9 cm gegossen. Ferner werden Kontrollplatten ohne Präparatbeimischung aufgestellt.

Ist der Nährboden erkaltet und fest, werden die Platten mit den in der Tabelle angegebenen Pilzarten beimpft und bei etwa 21°C inkubiert.

Die Auswertung erfolgt je nach Wachstumsgeschwindigkeit der Pilze nach 4—10 Tagen. Bei der Auswertung wird das radiale Myzelwachstum auf den behandelten Nährböden mit dem Wachstum auf dem Kontrollnährboden verglichen. Die Bonitierung des Pilzwachstums geschieht mit folgenden Kennzahlen:

1 kein Pilzwachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

Bei diesem Test zeigen z. B. die folgenden Verbindungen eine sehr gute Wirkung, die derjenigen der aus dem Stand der Technik bekannten Verbindung (B) überlegen ist:

Verbindungen gemäß Herstellungsbeispielen 1 und 2.

Tabelle E

Myzelwachstums-Test

| Wirkstoffe | Wirkstoffkonzentration ppm | Fusarium culmorum | Sclerotinia sclerotiorum | Fusarium nivale | Colletotrichum coffeanum | Rhizotonia solani | Pythium ultimum | Cochlobolus miyabeanus | Botrytis cinerea | Verticillium alboatrum | Pyricularia oryzae | Phialophora cinerescens | Helminthosporium gramineum | Mycosphaerella musocola | Phytophthora cactorum | Venturia inaequalis | Pellicularia sasakii | Xanthomonas oryzae |
|---|---|---|---|---|---|---|---|---|---|---|---|---|---|---|---|---|---|---|
| 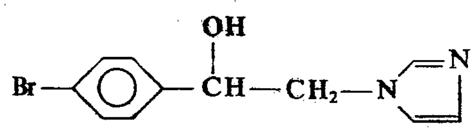 (B) (bekannt) | 10 | 9 | 9 | 9 | – | 9 | 9 | 9 | 9 | 9 | 9 | 9 | 9 | 9 | 9 | – | 9 | – |
| 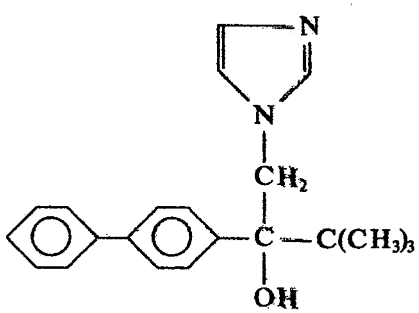 (1) | | 5 | 3 | 5 | – | 1 | 2 | 1 | 2 | 3 | 1 | 1 | 2 | 1 | 1 | – | 1 | – |
| 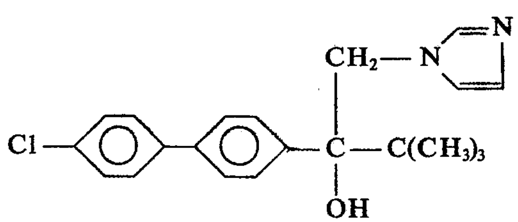 (2) | | 5 | 3 | 5 | – | 1 | 1 | 2 | 3 | 3 | 1 | 1 | 3 | 1 | 5 | – | 1 | – |

**Patentansprüche**

1. Hydroxybutyl-imidazol-Derivate der allgemeinen Formel

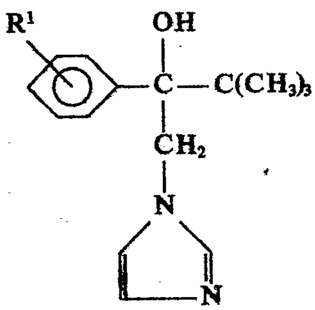

(I)

in welcher
$R^1$ für gegebenenfalls durch Fluor, Chlor, Brom, Alkyl und Alkoxy mit jeweils 1 bis 2 Kohlenstoffatomen substituiertes Phenyl steht

und deren physiologisch verträglichen Säureadditions-Salze.

2. Verfahren zur Herstellung von Hydroxybutyl-imidazol-Derivate, dadurch gekennzeichnet, daß man Oxirane der Formel

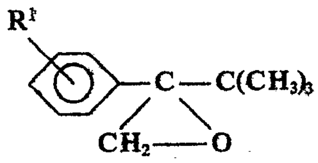

(II)

in welcher
$R^1$ die in Anspruch 1 angegebene Bedeutung hat,

mit Imidazol in Gegenwart eines Alkalialkoholats und in Gegenwart eines Verdünnungsmittels umsetzt.

3. Fungizide Mittel, gekennzeichnet durch einen Gehalt an mindestens einem Hydroxybutyl-imidazol-Derivat der Formel I in Anspruch 1.

4. Verwendung von Hydroxybutyl-imidazol-Derivaten der Formel I in Anspruch 1 zur Bekämpfung von Pilzen.

5. Oxirane der Formel

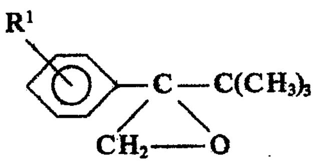

(II)

in welcher
$R^1$ die in Anspruch 1 angegebene Bedeutung hat.

**Claims**

1. Hydroxybutyl-imidazole derivatives of the general formula

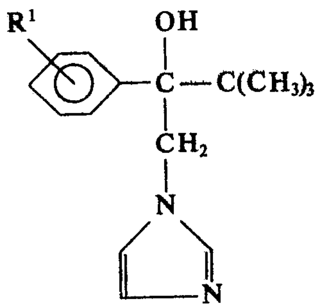

(I)

in which
$R^1$ represents phenyl which ist optionally substituted by fluorine, chlorine, bromine, or alkyl or alkoxy with in each case 1 to 2 carbon atoms,

and physiologically acceptable acid addition salts thereof.

2. Process for the preparation of hydroxybutyl-imidazole derivates, characterised in that oxiranes of the formula

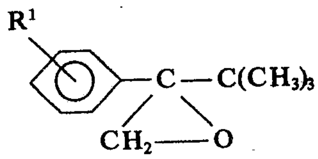

(II)

in which
$R^1$ has the meaning indicated in Claim 1,

are reacted with imidazole in the presence of an alkali metal alcoholate and in the presence of a diluent.

3. Fungicidal agents, characterised in that they contain at least one hydroxybutyl-imidazole derivate of the formula I in Claim 1.

4. Use of hydroxybutyl-imidazole derivatives of the formula I in Claim 1 for combating fungi.

5. Oxiranes of the formula

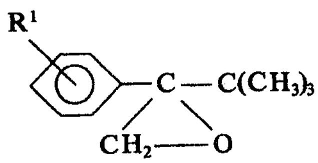

(II)

in which
$R^1$ has the meaning indicated in Claim 1.

**Revendications**

1. Dérivés d'hydroxybutyl-imidazole de formule générale

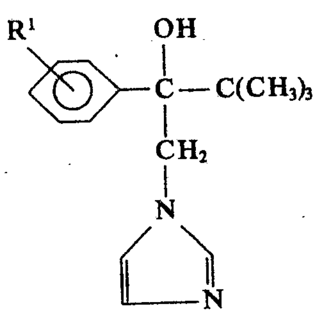

(I)

dans laquelle
$R^1$ est un groupe phényle éventuellement substitué par du fluor, du chlore, du brome, un radical alkyle et un radical alkoxy ayant chacun 1 ou 2 atomes de carbone,

et leurs sels d'addition d'acides physiologiquement acceptables.

2. Procédé de production de dérivés d'hydroxybutyl-imidazole, caractérisé en ce qu'on fait réagir des oxiranes de formule

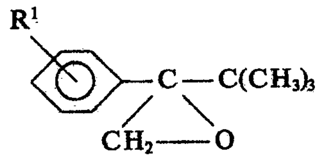

(II)

dans laquelle
$R^1$ a la définition indiquée dans la revendication 1,

avec l'imidazole en présence d'un alcoolate alcalin et en présence d'un diluant.

3. Compositions fongicides, caractérisées par une teneur en au moins un dérivé d'hydroxybutyl-imidazole de formule I suivant la revendication 1.

4. Utilisation de dérivés d'hydroxybutyl-imidazole de formule I suivant la revendication 1 pour combattre des champignons.

5. Oxiranes de formule

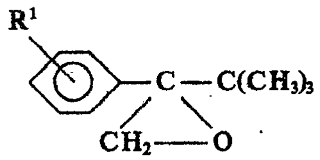

(II)

dans laquelle
$R^1$ a la définition indiquée dans la revendicátion 1.